# EUROPÄISCHE PATENTSCHRIFT

(11) **EP 0 997 188 B1**
(45) Veröffentlichungstag und Bekanntmachung des Hinweises auf die Patenterteilung: **24.03.2004**
(21) Anmeldenummer: 99120941.2
(22) Anmeldetag: 02.11.1999
(51) Int. Cl.: B01J 19/32, B01J 19/30, B01J 35/02, B01D 53/18, C07C 231/14, C07C 233/05

(54) **Packungselement**
Packing element
Elément de garnissage

(30) Priorität: 30.10.1998 DE 19850141
(43) Veröffentlichungstag der Anmeldung: 03.05.2000
(73) Patentinhaber: BASF AKTIENGESELLSCHAFT, 67056 Ludwigshafen (DE)
(72) Erfinder: Winter, Manfred, 67596 Dittelsheim-Hessloch (DE)
(74) Vertreter: Isenbruck, Günter, Dr.

(56) Entgegenhaltungen:
- WO-A-86/03822
- WO-A-96/33017
- DE-U- 1 426 310
- US-A- 4 122 011
- US-A- 5 417 938

## Beschreibung

Heterogen katalysierte Pyrolysereaktionen, wie die Pyrolyse von Formylalaninnitril (FAN) zu Vinylformamid und Blausäure, werden üblicherweise an losen Schüttungen von mit der katalytisch aktiven Substanz beschichteten Katalysatorfüllkörpern durchgeführt. Solche Füllkörperschüttungen weisen den Nachteil eines relativ großen Druckverlustes auf. Trotz gleicher Füllhöhe der Katalysatorschüttung können die Druckverluste unterschiedlich groß sein, so daß sich beim Einsatz von Mehrrohrreaktoren unter Umständen unterschiedliche Druckverluste in den einzelnen Reaktionsrohren ergeben können. Dies kann zur Ungleichheit von Temperatur und Verweilzeit der Reaktanten in den einzelnen Reaktionsrohren führen, welche sich auf den Reaktionsumsatz und die Reaktionsausbeute auswirken können.

Es ist bekannt, daß bei Temperaturen > 350°C der Energietransport durch Strahlung gegenüber der thermischen Wärmeleitung in Gasen stark zunimmt, so daß bei diesen Temperaturen ein hoher Anteil der Wärmeübertragung durch Strahlung erfolgt. Füllkörperschüttungen weisen jedoch oft nur eine geringe Strahlungsdurchlässigkeit auf, so daß sich im Reaktionsrohr ein ausgeprägtes radiales Temperaturprofil ergeben kann. Dieses Problem konnte durch Einsatz weitgehend strahlungsdurchlässiger Füllkörper, beispielsweise sogenannter Hiflow-Ringe, nur teilweise gelöst werden. Eine ungleiche radiale Temperaturverteilung ergibt sich zum Teil schon dadurch, daß die Strahlungsintensität mit zunehmendem Strahlungsweg abnimmt. Entsprechend ist bei exothermen Reaktionen die Temperatur im Reaktorkern am höchsten und in unmittelbarer Nähe der Reaktorwand am niedrigsten, bei endothermen Reaktionen stellen sich umgekehrte Temperaturverhältnisse ein. Unterschiedliche Temperaturen bedingen jedoch unterschiedliche Reaktionsgeschwindigkeiten mit der Folge, daß dem radialen Temperaturprofil entsprechende radiale Konzentrationsprofile der Edukt- bzw. Produktkonzentration auftreten können, die sich negativ auf die Ausbeute der Reaktion auswirken.

Reaktormaterial und Füllkörpermaterial haben unterschiedliche thermische Ausdehnungskoeffizienten. Beim Aufheizen dehnt sich das Reaktionsrohr aus, die Katalysatorschüttung setzt sich in dem Rohr und wird beim Abkühlen des Rohres von diesem teilweise zerdrückt. Dieser Katalysatorbruch führt zu einer Erhöhung des Druckverlustes, so daß die Schüttung häufig gewechselt werden muß.

Füllkörper aus zerbrechlichem Material können zudem nur in einen mit Wasser gefluteten Reaktor eingebracht werden, so daß das Befüllen des Reaktors mit dem Katalysator und der Austausch der Katalysatorschüttung aufwendig sind. Bei mit wasserlöslichen Substanzen dotierten Trägerkatalysatoren, wie den für die Formylalaninnitril-Pyrolyse verwendeten, mit Kaliumcarbonat dotierten Al₂O₃-Trägerkatalysatoren, kann die Dotierung erst nach dem Befüllen des Reaktors mit den Katalysatorfüllkörpern in dem Reaktor erfolgen, wobei vor und nach dem Imprägnieren getrocknet werden muß. Diese Vorgehensweise ist extrem aufwendig.

Aufgabe der Erfindung ist es, Katalysatorformkörper bereitzustellen, die die oben genannten Nachteile nicht aufweisen.

Die Aufgabe wird erfindungsgemäß gelöst durch einen Formkörper für chemische Apparate, der aus mindestens zwei geschlossenen Rahmen und einem oder mehreren diese Rahmen verbindenden Stegen gebildet wird, wobei die Rahmen voneinander beabstandet in einer Ebene oder mehreren parallelen Ebenen um eine (gedachte) Formkörperachse, die zu der bzw. den Ebenen senkrecht ist und durch die Mittelpunkte der Rahmen verläuft, angeordnet sind.

Die Rahmen können in einer Ebene oder mehreren parallelen Ebenen angeordnet sein, wobei mehrere Rahmen, die in der gleichen Ebene angeordnet sind, voneinander beabstandet und ineinander liegend angeordnet sind. In einer Ausführungsform wird der Formkörper aus mindestens drei Rahmen gebildet, wobei die Rahmen in mindestens zwei parallelen Ebenen angeordnet sind. Beispielsweise kann eine Ebene zwei ineinander liegende Rahmen und eine weitere Ebene nur einen Rahmen aufweisen.

Bevorzugt ist ein Formkörper für chemische Apparate, der aus mindestens vier geschlossenen Rahmen und einem oder mehreren diese Rahmen verbindenden Stegen gebildet wird, wobei die Rahmen voneinander beabstandet in mindestens zwei parallelen Ebenen um eine (gedachte) Formkörperachse, die zu den Ebenen senkrecht ist und durch die Mittelpunkte der Rahmen verläuft, ineinander liegend angeordnet sind und jede Ebene mindestens zwei Rahmen aufweist.

Chemische Apparate sind beispielsweise Rohrreaktoren, Rohrbündelreaktoren, Destillationskolonnen oder Gaswäscher. Die erfindungsgemäßen Formkörper können in diesen als Katalysatoren, Katalysatorträger oder Packungselemente, die den Wärme- und/oder Stoffaustausch fördern, eingesetzt werden.

Die geschlossenen Rahmen, Stege oder der gesamte Formkörper können kompakt, porös oder anders strukturiert sein. Beispielsweise können die Rahmen innen hohl sein. Die geschlossenen Rahmen können beliebige Geometrie aufweisen.

Die Rahmen können kreisrunde Geometrie aufweisen. Geschlossene Rahmen mit kreisrunder Geometrie werden auch als Ringe bezeichnet. Geschlossene Rahmen mit anderer als kreisrunder Geometrie sind somit Ringen entsprechende Gebilde, welche statt der kreisrunden eine andere Geometrie aufweisen.

Die Rahmen können eine Geometrie aufweisen, die ausschließlich durch kurvenförmige Linien begrenzt wird. Neben kreisrunder Geometrie können die Rahmen beispielsweise eine ovale Geometrie aufweisen.

Die Rahmen können Kanten und Ecken aufweisen. Die Rahmen können beispielsweise die Geometrie von Polygonen aufweisen. Polygone sind Dreiecke, Vierecke, Fünfecke usw..

Die Rahmen können beispielsweise sternförmige Geometrie oder die Geometrie einer Rosette (kreisförmig geschlossene Wellenlinie) aufweisen.

Die Rahmen weisen vorzugsweise im wesentlichen die Ausdehnung des Rohrquerschnittes auf, wobei ein gewisses Spiel beim Einbringen des Formkörpers in das Reaktionsrohr erforderlich ist. Durch entsprechende Wahl der Rahmengeometrie kann der Formkörper an den Querschnitt des Reaktionsrohres angepaßt werden.

Im allgemeinen, aber nicht zwingend, weisen Reaktionsrohre einen kreisrunden Querschnitt auf. Daher sind Rahmengeometrien bevorzugt, deren Umfang durch einen Kreis begrenzt wird. Bevorzugte Rahmen mit polygonaler Geometrie sind daher solche, deren Ecken auf einer Kreisline liegen.

Besonders bevorzugte Rahmen weisen eine hohe Symmetrie auf. Besonders bevorzugte Rahmen mit polygoner Geometrie weisen daher die Geometrie von regelmäßigen Polygonen auf Regelmäßige Polygone sind Quadrate sowie Dreiecke, Fünfecke, Sechsecke usw. mit gleich langen Seiten. Davon sind insbesondere solche Rahmen bevorzugt, die mindestens 5 Seiten aufweisen.

Insbesondere bevorzugt sind Rahmen mit kreisrunder Geometrie (Ringe).

In einer bevorzugten Ausführungsform der erfindungsgemäßen Formkörper sind die Rahmen benachbarter Ebenen bezüglich einer (gedachten) Parallelverschiebung entlang der gemeinsamen Achse der Rahmen nicht deckungsgleich. Die Deckungsungleichheit der Rahmen wirkt der Ausbildung laminarer Strömungsprofile im Reaktionsrohr entgegen und fördert damit den Stoffaustausch in radialer Richtung.

Nicht deckungsgleich sind die Rahmen beispielsweise, wenn sie unterschiedliche Formen aufweisen. So kann eine Ebene sechseckige Rahmen und die darüber bzw. darunter liegende Ebene fünfeckige oder siebeneckige Rahmen aufweisen. Nicht deckungsgleich sind die Rahmen auch, wenn sie zwar gleiche Geometrie und Größe aufweisen, aber in den Ebenen jeweils unterschiedlich orientiert sind. Beispielsweise können benachbarte Ebenen Rahmen von regelmäßig sechseckiger Geometrie aufweisen, die um einen Winkel zwischen 1° und 59° gegeneinander verdreht sind.

Nicht deckungsgleich sind die Rahmen auch, wenn sie zwar gleiche Geometrie und Orientierung, aber unterschiedliche Größe aufweisen. In einer bevorzugten Ausführungsform der erfindungsgemäßen Formkörper sind Rahmen gleicher Geometrie, aber abnehmender Größe, wobei es keine zwei Rahmen gibt, die die gleiche Größe aufweisen, in jeweils zwei Ebenen gleich orientiert, aber in der Reihenfolge ihrer Größe abwechselnd in der oberen und der unteren der beiden Ebenen angeordnet. Besonders bevorzugt ist eine Anordnung, in der die Rahmen in benachbarten Ebenen auf Lücke angeordnet sind, sich die Größe der Rahmen also so unterscheidet, daß ein Rahmen einer Ebene oberhalb bzw. unterhalb der Lücke angeordnet ist, die aus dem nächstgrößeren und nächstkleineren Rahmen der darüber- bzw. darunterliegenden Ebene gebildeten wird.

Die erfindungsgemäßen Formkörper weisen pro Ebene mindestens zwei Rahmen auf Die Formkörper können in jeder Ebene die gleiche Anzahl oder eine unterschiedliche Anzahl Rahmen aufweisen, bevorzugt weisen sie in jeder Ebene die gleiche Anzahl an Rahmen auf. Besonders bevorzugt weisen sie in jeder Ebene mindestens drei, insbesondere mindestens vier Rahmen auf.

Die Abstände zwischen den Rahmen einer Ebene mit drei oder mehr Rahmen können gleich oder verschieden sein. In einer bevorzugten Ausführungsform sind die Abstände zwischen den Rahmen gleich groß. Durch Formkörper mit äquidistanten Rahmen wird in einem Strömungsreaktor über den Querschnitt des Reaktionsrohres verteilt ein gleichbleibender Druckverlust hervorgerufen. Dadurch sind die Strömungsgeschwindigkeit und Verweilzeit im Zentrum und in den Randzonen des Reaktionsrohres im wesentlichen gleich groß.

In einer weiteren bevorzugten Ausführungsform nimmt der Abstand der Rahmen einer Ebene von außen nach innen ab. Solche Formkörper bewirken im Zentrum des Reaktionsrohres einen höheren Druckverlust als in den Randbereichen. Damit ist im Zentrum des Rohres die Strömungsgeschwindigkeit geringer und die Verweilzeit größer als in den Randbereichen des Rohres.

In einer weiteren bevorzugten Ausführungsform nimmt der Abstand der Rahmen einer Ebene von außen nach innen zu. Solche Formkörper bewirken im Zentrum des Reaktionsrohres einen geringeren Druckverlust als in den Randbereichen. Damit ist im Zentrum des Rohres die Strömungsgeschwindigkeit höher und die Verweilzeit geringer als in den Randbereichen des Rohres.

Durch entsprechende Wahl der Abstände zwischen den Rahmen einer Ebene kann damit ein Verweilzeitspektrum eingestellt werden, welches die Wirkung eines radialen Temperaturprofils auf den Reaktionsfortschritt an unterschiedlichen Stellen des Reaktorquerschnitts ausgleicht. So kann bei exothermen Reaktionen, da die Wärmeableitung über die Rohrwand erfolgt, die Temperatur im Zentrum des Reaktionsrohres höher als in den Randbereichen sein. Bei Reaktionen mit positiver Aktivierungsenergie ist dann auch die Reaktionsgeschwindigkeit im Zentrum höher. In diesem Fall bewirkt der Einsatz von erfindungsgemäßen Formkörpern mit von außen nach innen zunehmenden Abständen zwischen den Rahmen, daß die im Zentrum höhere Reaktionsgeschwindigkeit durch eine kürzere Verweilzeit der Reaktanten zumindest teilweise ausgeglichen wird, wodurch der Bildung radialer Konzentrationsprofile entgegengewirkt wird. Umgekehrt kann bei endothermen Reaktionen, da die Wärmezufuhr über die Rohrwand erfolgt, die Temperatur im Zentrum des Reaktionsrohres niedriger als in den Randbereichen sein. Bei Reaktionen mit Aktivierungsenergie ist dann die Reaktionsgeschwindigkeit im Zentrum niedriger. In diesem Fall bewirkt der Einsatz von Formkörpern mit von außen nach innen abnehmenden Abständen zwischen den Rahmen, daß die im Zentrum niedrigere Reaktionsgeschwindigkeit durch eine längere Verweilzeit der Reaktanten zumindest teilweise ausgeglichen wird.

Bei Reaktionen, deren Reaktionsgeschwindigkeit im wesentlichen temperaturunabhängig ist, kann der Einsatz von Formkörpern mit äquidistanten Rahmen vorteilhaft sein.

Einer vom Rand zum Zentrum des Reaktionsrohres hin ansteigenden Temperatur und der dadurch bewirkten Erhöhung der Reaktionsgeschwindigkeit wird auch dadurch entgegengewirkt, daß bei Formkörpern mit von außen nach innen zunehmendem Rahmenabstand - bei gleichbleibendem Umfang des Rahmenquerschnitts - die gesamte Rahmenoberfläche pro Reaktorvolumen von außen nach innen abnimmt. Damit nimmt bei mit einer katalytisch aktiven Substanz beschichteten Rahmen die Kontaktfläche und damit die Aktivität des Katalysators von außen nach innen ab. Ferner wird Wärmestrahlung von den in den Randbereichen des Reaktionsrohres geringer beabstandeten, also dichter angeordneten Rahmen wegen der dort größeren Angriffsfläche für Wärmestrahlung stärker absorbiert als im Zentrum des Reaktionsrohres, wodurch ein insgesamt ausgeglicheneres Temperaturprofil erreicht werden kann.

Der durch eine vom Rand zum Zentrum des Reaktionsrohres hin abfallenden Temperatur bewirkten Erniedrigung der Reaktionsgeschwindigkeit wird entsprechend dadurch entgegengewirkt, daß bei Formkörpern mit von außen nach innen abnehmendem Abstand der Rahmen - bei gleichbleibendem Rahmenquerschnitt - - die gesamte Rahmenoberfläche pro Reaktorvolumen von außen nach innen zunimmt. Damit nimmt bei mit einer katalytisch aktiven Substanz beschichteten Rahmen die Kontaktfläche und damit die Aktivität des Katalysators von außen nach innen zu. Ferner wird Wärmestrahlung von den in den Randbereichen des Reaktionsrohres stärker beabstandeten, also weniger dicht angeordneten Rahmen wegen der dort geringeren Angriffsfläche für Wärmestrahlung weniger stark absorbiert als im Zentrum des Reaktionsrohres, wodurch ein insgesamt ausgeglicheneres Temperaturprofil erreicht werden kann.

In einer besonders bevorzugten Ausführungsform nimmt der Abstand zwischen den Rahmen einer Ebene in jeder Ebenen von außen nach innen entweder zu oder ab.

In einer weiteren bevorzugten Ausführungsform der Formkörper nimmt der Umfang des Rahmenquerschnitts von außen nach innen entweder zu oder ab, wobei besonders bevorzugt auch die Ausdehnung des Rahmenquerschnitts in Richtung der Formkörperachse zu- oder abnimmt. Durch einen zunehmenden Umfang des Rahmenquerschnitts wird die Kontaktfläche des Katalysators bzw. die Angriffsfläche für Wärmestrahlung pro Reaktorvolumen erhöht. Eine Zu- bzw. Abnahme des Umfangs des Rahmenquerschnitts von außen nach innen wirkt also genauso wie eine Ab- bzw. Zunahme des Rahmenabstands von außen nach innen.

In einer weiteren bevorzugten Ausführungsform weisen die Formkörper äquidistante Rahmen mit von außen nach innen zunehmendem Umfang des Rahmenquerschnitts auf. Solche Formkörper werden vorteilhaft zur Durchführung endothermer Reaktionen eingesetzt. In einer weiteren bevorzugten Ausführungsform weisen die Formkörper äquidistante Rahmen mit von außen nach innen abnehmendem Umfang des Rahmenquerschnitts auf. Solche Formkörper werden vorteilhaft zur Durchführung exothermer Reaktionen eingesetzt.

In einer besonders bevorzugten Ausführungsform weisen die Formkörper Rahmen mit von außen nach innen abnehmendem Abstand der Rahmen und zunehmendem Umfang des Rahmenquerschnitts auf. Solche Formkörper werden besonders vorteilhaft zur Durchführung endothermer Reaktionen eingesetzt. In einer weiteren besonders bevorzugten Ausführungsform weisen die Formkörper Rahmen mit von außen nach innen zunehmendem Abstand der Rahmen und abnehmendem Umfang des Rahmenquerschnitts auf. Solche Formkörper werden besonders vorteilhaft zur Durchführung exothermer Reaktionen eingesetzt.

Die Rahmen können einen Querschnitt beliebiger Form aufweisen. Im allgemeinen hat der Querschnitt der Rahmen eine einfache Form, vorzugsweise die eines Kreises, einer Ellipse, eines Tropfens, eines Eis, eines Rechtecks mit abgerundeten Ecken, eines Dreiecks, eines Parallelogramms, einer Raute, eines Rechtecks oder Quadrats, oder eines Polygons mit fünf und mehr Seiten.

Ein Rechteck mit abgerundeten Ecken hat im Extremfall eine Form, die man sich aus einem Rechteck und auf gegenüberliegenden Seiten dieses Rechtecks aufgesetzten Halbkreisen mit einem der Seitenlänge entsprechenden Durchmesser zusammengesetzt denken kann. Rechtecke mit abgerundeten Ecken sind auch alle Übergangsformen zwischen dieser Extremform und einem Rechteck.

In einer bevorzugten Ausführungsform weisen die Querschnitte der Rahmen eine längliche Form mit voneinander unterscheidbarer Längs- und Querachse auf Beispiele sind Querschnittsflächen in der Form einer Ellipse, eines Eis, eines Rechtecks mit abgerundeten Ecken, eines Rechtecks (mit Ausnahme eines Quadrats), einer Raute oder eines Parallelogramms. In den beispielhaft genannten Fällen symmetrischer Querschnittsflächen liegt die Längsachse in der Symmetrieebene der Flächen.

Die Rahmen können alle den gleichen oder verschiedene Querschnitte aufweisen. Bei Formkörpern mit von außen nach innen zunehmendem (abnehmendem) Umfang der Querschnittsfläche können der äußerste (innerste) Rahmen einer Ebene kreisrunden Querschnitt und die weiter innen (weiter außen) liegenden Rahmen elliptischen oder eiförmigen Querschnitt oder den Querschnitt eines Rechtecks mit abgerundeten Ecken aufweisen, wobei die Ausdehnung des Rahmenquerschnitts in Richtung der Formkörperachse von außen nach innen zunimmt (abnimmt).

In einer weiteren bevorzugten Ausführungsform sind die Rahmenquerschnitte gegenüber der Formkörperachse geneigt. Geneigte Rahmenquerschnitte weisen einen länglichen Querschnitt mit einer Längsachse und einer Querachse auf, wobei die Längsachsen gegenüber der Formkörperachse um einen Winkel von 1 bis 45° geneigt ist. Durch die Neigung der Rahmenquerschnitte wird der Ausbildung laminarer Strömungen zusätzlich entgegengewirkt und die Durchmischung der Reaktionsgase zusätzlich gefördert.

Die Neigung der Rahmenquerschnitte kann regelmäßig oder unregelmäßig sein. Unregelmäßig ist die Neigung beispielsweise dann, wenn die Rahmenquerschnitte in unregelmäßiger Abfolge nach rechts und nach links geneigt sind.

Bevorzugt sind die Rahmenquerschnitte regelmäßig geneigt. Regelmäßig geneigt sind die Rahmenquerschnitte dann, wenn die Rahmenquerschnitte in regelmäßiger Abfolge, beispielsweise in einer Ebene alternierend nach rechts und nach links geneigt sind.

In einer besonders bevorzugten Ausführungsform der Formkörper sind in einer Schnittebene, welche die Formkörperachse enthält und die Rahmen senkrecht schneidet, auf jeweils einer Seite von der Formkörperachse die Rahmenquerschnitte in einer Ebene im gleichen Sinn (nach rechts bzw. nach links) geneigt und die Rahmenquerschnitte in benachbarten Ebenen im entgegengesetzten Sinn geneigt. Dabei können die Rahmenquerschnitte in einer Ebene auf entgegengesetzten Seiten der Formkörperachse im gleichen Sinn geneigt oder auf entgegengesetzten Seiten im entgegengesetzten Sinn geneigt sein. Die zuletzt beschriebene Ausführungsform kann beispielsweise durch die Verwendung konzentrischer, sich konusförmig verjüngender Ringe mit länglichem Querschnitt verwirklicht werden, die in einer Ebene mit der sich verjüngenden Seite nach unten und in den benachbarten Ebenen mit der sich verjüngenden Seite nach oben angeordnet sind.

Die Formkörper können grundsätzlich beliebig viele Ebenen aufweisen. Aus Gründen der einfacheren Herstellbarkeit werden die Formkörper im allgemeinen genau zwei Ebenen aufweisen.

Die erfindungsgemäßen Formkörper weisen die Rahmen verbindende Stege auf. Grundsätzlich sind Zahl und Anordnung der Stege beliebig, solange die Stege so angeordnet sind, daß sich eine ausreichende Stabilität der Formkörper ergibt. Vorzugsweise sind die Stege in axialer Richtung angeordnet. Die Stege können jeweils alle Rahmen oder nur einen Teil der Rahmen verbinden. Beispielsweise können die Formkörper zwei, drei, vier oder mehr vom äußersten zum innersten Rahmen durchlaufende Stege aufweisen, die jeweils alle Rahmen aller Ebenen verbinden. Die Formkörper können beispielsweise Stege aufweisen, wobei ein Teil der Stege jeweils nur die äußeren und mittleren Rahmen der Ebenen und ein anderer Teil nur die mittleren und inneren Rahmen der Ebenen verbinden. Die Stege können beispielsweise auch eine U-Form aufweisen, wobei die Schenkel des U nur jeweils die Ringe nur einer Ebene verbinden und die Basis des U benachbarte Ebenen verbindet.

In einer bevorzugten Ausführungsform weisen die Formkörper Stege auf, die jeweils alle Rahmen verbinden, und Stege auf, die jeweils nur einen Teil der Rahmen verbinden, wobei die Ausdehnung der Stege nach innen im wesentlichen durch den innersten Rahmen begrenzt wird. Durch die Begrenzung der Stege durch den innersten Rahmen können diese mittels einer durch die innersten Rahmen durchgreifenden Stange besonders leicht in einen Rohrreaktor eingebracht werden. Dadurch entfällt die Notwendigkeit, vor dem Einbringen der Formkörper in den Reaktor diesen mit Wasser zu füllen.

In einer besonders bevorzugten Ausführungsform wird die Ausdehnung der Formkörper in Richtung der Formkörperachse auf der Unterseite mit den Stegen und auf der Oberseite von den Rahmen begrenzt. Dadurch liegen im gestapelten Zustand die Formkörpern auf der Unterseite mit den Rahmen auf der Oberseite der Stege des jeweils darunterliegenden Formkörpers auf, wodurch eine gute Stapelbarkeit gewährleistet wird. Vorzugsweise sind die Abmessungen der Stege so, daß im gestapelten Zustand sich eine äquidistante Anordnung aller Ebenen aller Formkörper ergibt.

Die erfindungsgemäßen Formkörper können aus allen Werkstoffen, die auch zur Herstellung von Füllkörpem verwendet werden, hergestellt sein. Beispiele sind keramische Werkstoffe, wie Steinzeug, Porzellan und Sinterkorund, metallische Werkstoffe, wie C-Stahl, Edelstahl, Titan, Nickel, Kupfer, Kupfer und Aluminium oder Kunststoffe wie Polypropylen, Highdensity-Polyethylen, Polyvinylchlorid und Polyvinylidendifluorid.

Bei den erfindungsgemäßen Formkörpern tritt das Problem des Katalysatorbruchs durch Zerdrücken naturgemäß nicht auf.

Die erfindungsgemäßen Formkörper können als Packungselemente in Destillationskolonnen oder in Gaswäschern verwendet werden.

Bevorzugt werden die erfindungsgemäßen Formkörper als Katalysatorformkörper verwendet. Die als Katalysatorformkörper verwendeten erfindungsgemäßen Formkörper können dabei ganz aus beliebigen katalytisch aktiven Substanzen hergestellt sein oder mit beliebigen katalytisch aktiven Substanzen beschichtet sein. Sie können zur Katalyse beliebiger heterogen katalysierter Gasphasenreaktionen oder Flüssigphasenreaktionen eingesetzt werden.

Beispielsweise können aus Al₂O₃ hergestellte Formkörper, die mit Kaliumcarbonat imprägniert sind, als Katalysatorformkörper zur Pyrolyse von Formylalaninnitril zu Vinylformamid und Blausäure oder von Formamid zu Wasser und Blausäure in einem Rohrreaktor verwendet werden.
Gegenstand der vorliegenden Erfindung ist auch ein Verfahren zur Pyrolyse von Formylalaninnitril oder Formamid in einem Rohrreaktor in Gegenwart eines aus Al₂O₃ hergestellten, mit Kaliumcarbonat dotierten erfindungsgemäßen Formkörpers.

Die erfindungsgemäßen Formkörper können nach allen bekannten Verfahren zur Herstellung von Füllkörpern oder Packungselementen hergestellt werden. Beispielsweise können die Formkörper durch Spritzguß, Pulverspritzguß oder Preßverfahren, wie Preßsintern, hergestellt werden.

Die Formkörper können am Stück oder in Form von zwei oder mehreren zusammensetzbaren Segmenten hergestellt werden. Gegenstand der Erfindung sind somit auch erfindungsgemäße Formkörper, die aus zwei oder mehreren Segmenten zusammensetzbar sind.

Segmente wurden durch (gedachte) Schnitte vom Umfang zur Achse der Formkörper erhalten. Segmente sind beispielsweise Hälften, Drittel, Viertel usw. der Formkörper.

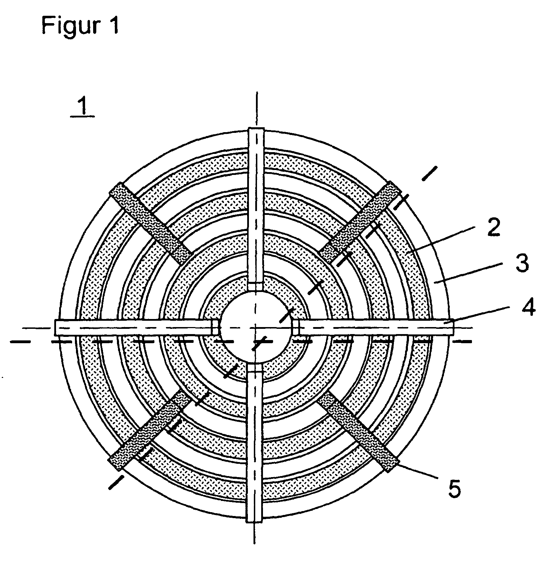

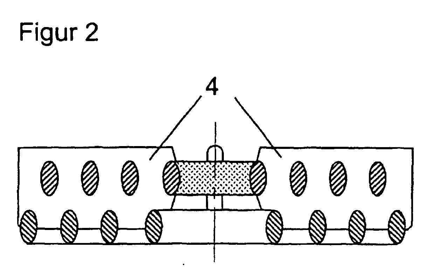

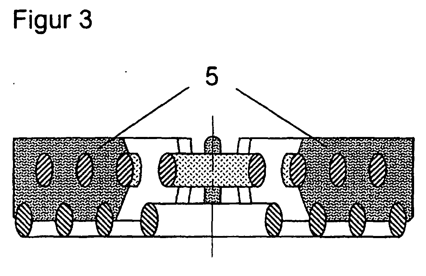

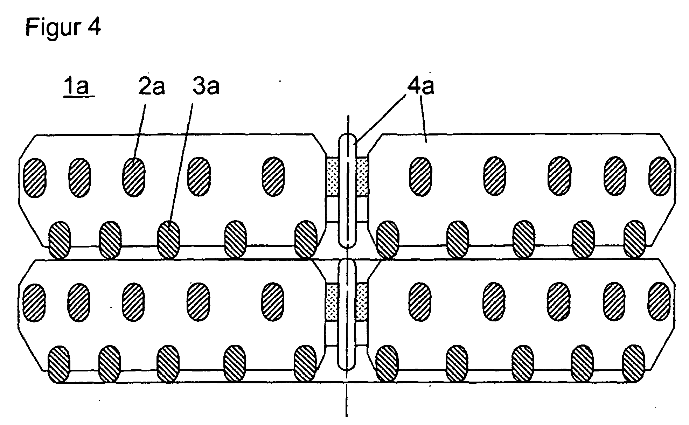

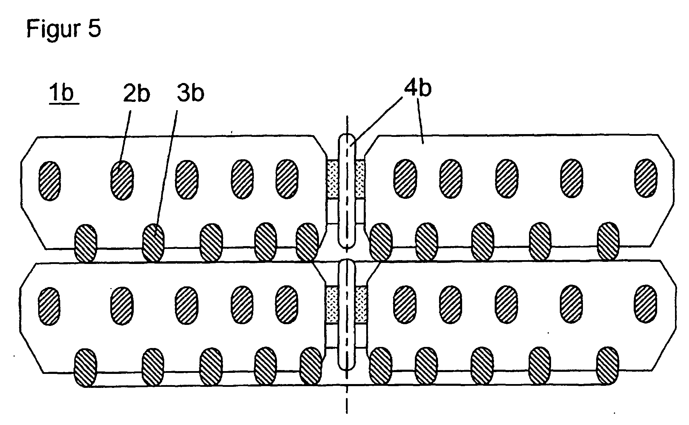

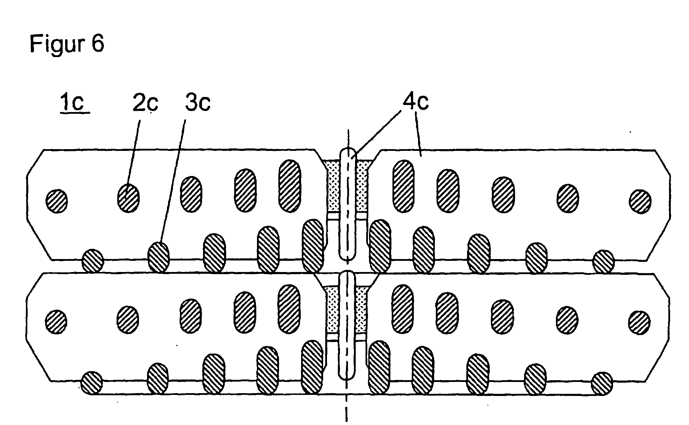

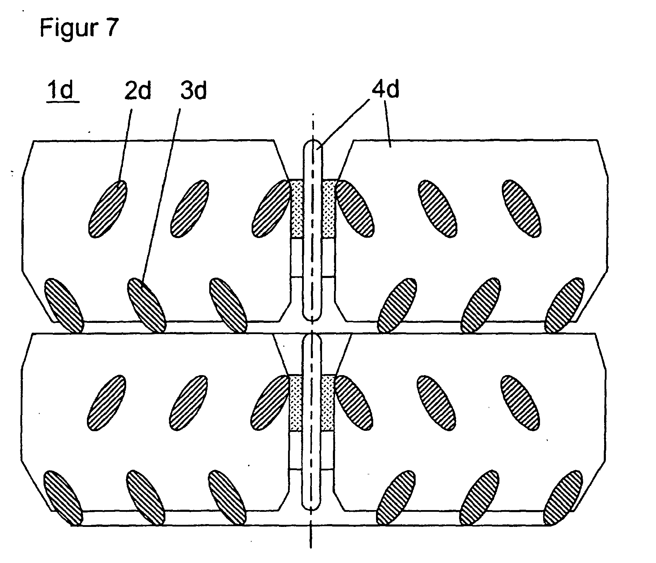

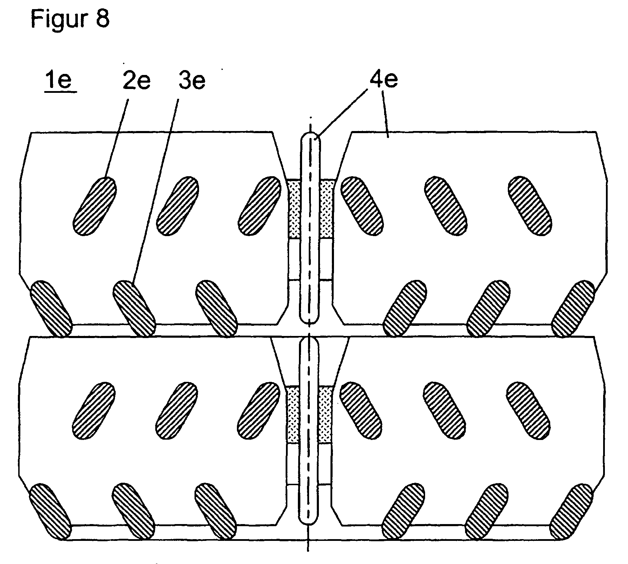

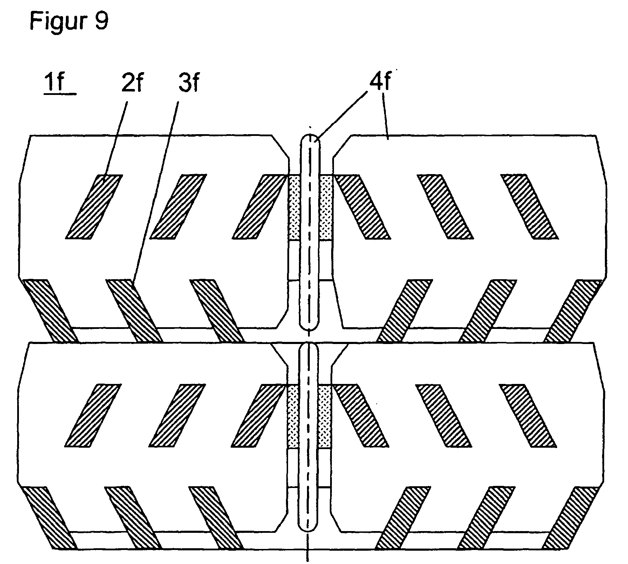

Die Erfindung wird durch die Figuren 1 bis 9, die spezielle Ausführungsformen der Formkörper zeigen, näher erläutert.
Figur 1 zeigt einen im ganzen mit (1) bezeichneten Formkörper in der Aufsicht. Der Formkörper (1) weist insgesamt vier in einer oberen Ebene angeordnete konzentrische Ringe (2) und insgesamt vier in einer unteren Ebene angeordnete konzentrische Ringe (3) mit elliptischem Querschnitt auf. Der Formkörper weist ferner insgesamt vier Stege (4), die jeweils alle Ringe beider Ebenen verbinden, sowie insgesamt vier Stege (5), welche jeweils die drei äußeren Ringe beider Ebenen verbinden, auf.
Figur 2 zeigt einen Schnitt durch einen Formkörper (1) entlang der Stege (4).
Figur 3 zeigt einen Schnitt durch den Formkörper (1) entlang der Stege (5).
Figur 4 zeigt einen Schnitt entlang der Stege (4a) durch zwei gestapelte, im ganzen mit (1a) bezeichneten weiteren Formkörper mit von außen nach innen zunehmend beabstandeten Rahmen (2a) und (3a). Der Rahmenquerschnitt weist die Form eines Rechtecks mit abgerundeten Ecken auf.
Figur 5 zeigt einen Schnitt entlang der Stege (4b) durch zwei gestapelte, im ganzen mit (1b) bezeichneten weiteren Formkörper mit von außen nach innen zunehmend beabstandeten Rahmen (2b) und (3b).
Figur 6 zeigt einen Schnitt entlang der Stege (4c) durch zwei gestapelte, im ganzen mit (1c) bezeichnete weitere Formkörper mit von außen nach innen abnehmend beabstandeten Rahmen (2c) und (3c) und von außen nach innen zunehmendem Umfang und axialer Ausdehnung des Rahmenquerschnitts.
Figuren 7 bis 9 zeigen jeweils Schnitte entlang der Stege (4d), (4e) bzw. (4f) durch im ganzen mit (1d), (1e) bzw. (1f) bezeichnete paarweise gestapelte weitere Formkörper mit Rahmen (2d), (2e) bzw. (2f) und (3d), (3e) bzw. (3f) mit länglichen, gegenüber der Formkörperachse in unterschiedlichem Sinn geneigten Querschnitten. Die Querschnitte der Rahmen (2d) und (3d), (2e) und (3e) bzw. (2f) und (3f) sind Beispiele für einen elliptischen, einen rechteckigen Querschnitt mit abgerundeten Ecken bzw. einen trapezförmigen Querschnitt.

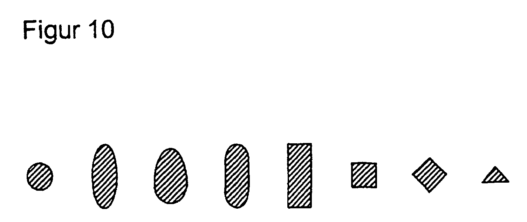

Figur 10 zeigt beispielhaft mögliche Rahmenquerschnitte. Diese weisen (von links nach rechts) eine kreisrunde, eine ovale, eine eiförmige, eine rechteckige Geometrie mit abgerundeten Ecken, eine rechteckige, eine quadratische, eine rautenförmige und eine dreieckig Geometrie auf.

## Patentansprüche

1. Stapelbares Packungselement für Rohrreaktoren, Destillationskolonnen und Gaswäscher, das aus mindestens vier geschlossenen Rahmen und einem oder mehreren diese Rahmen verbindenden Stegen gebildet wird, wobei die Rahmen voneinander beabstandet in mindestens zwei parallelen Ebenen um eine (gedachte) Formkörperachse, die zu den Ebenen senkrecht ist und durch die Mittelpunkte der Rahmen verläuft, ineinander liegend angeordnet sind und jede Ebene mindestens zwei Rahmen aufweist.

2. Stapelbares Packungselement nach Anspruch 1, **dadurch gekennzeichnet, daß** die Rahmen benachbarter Ebenen bezüglich einer (gedachten) Parallelverschiebung entlang der Formkörperachse nicht deckungsgleich sind.

3. Stapelbares Packungselement nach Anspruch 1 oder 2, das in jeweils zwei benachbarten Ebenen gleich orientiert angeordnete Rahmen gleicher Geometrie aufweist, wobei es keine zwei Rahmen gibt, welche die gleiche Größe aufweisen, und die Rahmen in der Reihenfolge ihrer Durchmesser abwechselnd in der oberen und der unteren der beiden benachbarten Ebenen angeordnet sind.

4. Stapelbares Packungselement nach einem der Ansprüche 1 bis 3, **dadurch gekennzeichnet, daß** jede Ebene mindestens drei Rahmen aufweist, wobei der Abstand zwischen den Rahmen einer Ebene in jeder Ebenen von außen nach innen entweder zunimmt oder abnimmt.

5. Stapelbares Packungselement nach einem der Ansprüche 1 bis 4, **dadurch gekennzeichnet, daß** der Umfang der Rahmenquerschnitte von außen nach innen entweder zunimmt oder abnimmt.

6. Stapelbares Packungselement nach einem der Ansprüche 1 bis 5, **dadurch gekennzeichnet, daß** die Rahmen kreisrunde Geometrie oder polygonale Geometrie aufweisen.

7. Stapelbares Packungselement nach einem der Ansprüche 1 bis 6, **dadurch gekennzeichnet, daß** die Rahmenquerschnitte die Form eines Kreises, einer Ellipse, eines Tropfens, eines Eis, eines Rechtecks mit abgerundeten Ecken, eines Dreiecks, eines Parallelogramms, einer Raute, eines Rechtecks, eines Quadrats oder eines Polygons mit fünf oder mehr Seiten aufweisen.

8. Stapelbares Packungselement nach einem der Ansprüche 1 bis 7, **dadurch gekennzeichnet, daß** die Rahmen einen länglichen Querschnitt mit einer Längsachse und einer Querachse aufweisen, wobei die Längsachsen gegenüber der Formkörperachse um einen Winkel von 1 bis 89° geneigt sind.

9. Stapelbares Packungselement nach Anspruch 8 mit mindestens zwei Ebenen, **dadurch gekennzeichnet, daß** in einer Schnittebene, welche die Formkörperachse enthält und die Rahmen senkrecht schneidet, auf jeweils einer Seite von der Formkörperachse die Rahmenquerschnitte in einer Ebene im gleichen Sinn geneigte Längsachsen und die Rahmenquerschnitte in benachbarten Ebenen im entgegengesetzten Sinn geneigte Längsachsen aufweisen.

10. Stapelbares Packungselement nach einem der Ansprüche 1 bis 9, **dadurch gekennzeichnet, daß** es Stege aufweist, die jeweils alle Rahmen verbinden, und Stege aufweist, die jeweils nur einen Teil der Rahmen verbinden, wobei die Ausdehnung der Stege nach innen im wesentlichen durch den innersten Rahmen begrenzt wird.

11. Stapelbares Packungselement nach einem der Ansprüche 1 bis 10, **dadurch gekennzeichnet, daß** dessen Ausdehnung in Richtung der Formkörperachse auf der einen Seite von den Stegen und auf der anderen Seite von den Rahmen begrenzt wird.

12. Stapelbares Packungselement nach einem der Ansprüche 1 bis 11, **dadurch gekennzeichnet, daß** es aus zwei oder mehreren Segmenten zusammensetzbar ist.

13. Stapelbares Packungselement nach einem der Ansprüche 1 bis 12, **dadurch gekennzeichnet, daß** es ganz aus einer katalytisch aktiven Substanz hergestellt ist oder mit einer katalytisch aktiven Substanz beschichtet ist.

14. Verwendung eines stapelbaren Packungselementes, wie es in Anspruch 13 definiert ist, für die Pyrolyse von Formylalaninnitril oder Formamid.

15. Verfahren zur Pyrolyse von Formylalaninnitril oder Formamid in einem Rohrreaktor, **dadurch gekennzeichnet, daß** die Pyrolyse in Gegenwart einer Packung von aus Al₂O₃ hergestellten, mit Kaliumcarbonat imprägnierten stapelbaren Packungselementen, wie sie in einem der Ansprüche 1 bis 13 definiert sind, erfolgt.

16. Packung aus gestapelkn Packungselementen, wie sie in einem der Ansprüche 1 bis 13 definiert sind.

17. Rohrreaktor, Destillationskolonne oder Gaswäscher, enthaltend eine Packung nach Anspruch 16.

## Claims

1. A stackable packing element for tubular reactors, distillation columns and gas scrubbers which is formed from at least four closed frames and one or more cross-pieces connecting these frames, where the frames are arranged one inside the other at a distance from one another in at least two parallel planes about an (imaginary) molding axis which is perpendicular to the planes and passes through the center points of the frames, and each plane has at least two frames.

2. A stackable packing element as claimed in claim 1, wherein the frames of adjacent planes are not congruent with respect to an (imaginary) parallel shift along the molding axis.

3. A stackable packing element as claimed in one of claims 1 and 2 which has frames of identical geometry which are arranged with the same orientation in each of two adjacent planes, with no two frames having the same size, and the frames are arranged in the sequence of their diameters alternately in the upper and lower of the two adjacent planes.

4. A stackable packing element as claimed in one of claims 1 to 3, wherein each plane has at least three frames, where the separation between the frames in a plane either increases or decreases from the outside inward in each plane.

5. A stackable packing element as claimed in one of claims 1 to 4, wherein the circumference of the frame cross sections either increases or decreases from the outside inward.

6. A stackable packing element as claimed in one of claims 1 to 5, wherein the frames have a circular geometry or a polygonal geometry.

7. A stackable packing element as claimed in one of claims 1 to 6, wherein the frame cross sections have the shape of a circle, an ellipse, a drop, an egg, a rectangle having rounded corners, a triangle, a parallelogram, a diamond, a rectangle, a square or a polygon having five or more sides.

8. A stackable packing element as claimed in one of claims 1 to 7, wherein the frames have an oblong cross section with a longitudinal axis and a transverse axis, where the longitudinal axes are inclined by an angle of from 1 to 89° with respect to the molding axis.

9. A stackable packing element as claimed in claim 8 having at least two planes, wherein, in a section plane containing the molding axis and cutting the frames perpendicularly, the frame cross sections in one plane have, in each case on one side of the molding axis, longitudinal axes which are inclined in the same direction and the frame cross sections in adjacent planes have longitudinal axes which are inclined in the opposite direction.

10. A stackable packing element as claimed in one of claims 1 to 9 which has cross-pieces which each connect all frames and cross-pieces which each connect only some of the frames, the extension of the cross-pieces to the inside being essentially delimited by the innermost frames.

11. A stackable packing element as claimed in one of claims 1 to 10 whose extension in the direction of the molding axis is delimited on one side by the cross-pieces and on the other side by the frames.

12. A stackable packing element as claimed in one of claims 1 to 11 which can be assembled from two or more segments.

13. A stackable packing element as claimed in one of claims 1 to 12 which is made entirely of a catalytically active substance or is coated with a catalytically active substance.

14. The use of a stackable packing element defined as in claim 13 for the pyrolysis of formylalanine nitrile or formamide.

15. A process for the pyrolysis of formylalanine nitrile or formamide in a tubular reactor, which comprises carrying out the pyrolysis in the presence of a packing from stackable packing elements as defined in one of claims 1 to 13 that have been prepared from Al₂ O₃ and impregnated with potassium carbonate.

16. A packing of stacked packing elements as defined in one of claims 1 to 13.

17. A tubular reactor, distillation column or gas scrubber containing a packing as claimed in claim 16.

## Revendications

1. Elément de garnissage empilable pour réacteurs tubulaires, colonnes de distillation et laveurs de gaz, qui est formé d'au moins quatre cadres fermés et d'une ou de plusieurs nervures reliant ces cadres, les cadres étant écartés les uns des autres en au moins deux plans parallèles autour d'un axe de corps formé (imaginaire) qui est vertical par rapport aux plans et passe par les centres des cadres, en étant incidents et chaque plan présentant au moins deux cadres.

2. Elément de garnissage empilable selon la revendication 1, **caractérisé en ce que** les cadres de plans adjacents ne sont pas coïncidents en ce qui concerne un déplacement parallèle (imaginaire) le long de l'axe de corps formé.

3. Elément de garnissage empilable selon la revendication 1 ou 2, qui présente des cadres de même géométrie, orientés de manière identique, dans respectivement deux plans adjacents, deux cadres qui présentent la même taille n'existant pas et les cadres étant placés en alternance dans le plan supérieur et dans le plan inférieur des deux plans adjacents, dans l'ordre de leur diamètre.

4. Elément de garnissage empilable, selon l'une quelconque des revendications 1 à 3, **caractérisé en ce que** chaque plan présente au moins trois cadres, l'écart entre les cadres d'un plan augmentant ou diminuant de l'extérieur vers l'intérieur dans chaque plan.

5. Elément de garnissage empilable, selon l'une quelconque des revendications 1 à 4, **caractérisé en ce que** la circonférence des sections de cadre augmente ou diminue de l'extérieur vers l'intérieur.

6. Elément de garnissage empilable, selon l'une quelconque des revendications 1 à 5, **caractérisé en ce que** les cadres présentent une géométrie circulaire ou polygonale.

7. Elément de garnissage empilable, selon l'une quelconque des revendications 1 à 6, **caractérisé en ce que** les sections de cadre présentent la forme d'un cercle, d'une ellipse, d'une goutte, d'une glace, d'un rectangle aux coins arrondis, d'un triangle, d'un parallélogramme, d'un losange, d'un carré ou d'un polygone à cinq côtés ou plus.

8. Elément de garnissage empilable, selon l'une quelconque des revendications 1 à 7, **caractérisé en ce que** les cadres présentent une section oblongue avec un axe longitudinal et un axe transversal, les axes longitudinaux étant inclinés d'un angle de 1 à 89° par rapport à l'axe de corps formé.

9. Elément de garnissage empilable, selon la revendication 8, avec au moins deux plans, **caractérisé en ce que** dans un plan de coupe, lequel comprend l'axe de corps formé et coupe les cadres verticalement, les sections de cadre dans un plan présentent des axes longitudinaux inclinés dans le même sens et les sections de cadre dans des plans adjacents présentent des axes longitudinaux inclinés en sens inverse, respectivement d'un côté de l'axe de corps formé.

10. Elément de garnissage empilable, selon l'une quelconque des revendications 1 à 9, **caractérisé en ce qu'**il présente des nervures qui relient respectivement tous les cadres, et qu'il présente des nervures qui relient respectivement seulement une partie des cadres, l'étendue des cadres vers l'intérieur étant limitée essentiellement par le cadre le plus à l'intérieur.

11. Elément de garnissage empilable, selon l'une quelconque des revendications 1 à 10, **caractérisé en ce que** son étendue en direction de l'axe de corps formé est limitée d'un côté par les nervures et de l'autre côté par les cadres.

12. Elément de garnissage empilable, selon l'une quelconque des revendications 1 à 11, **caractérisé en ce qu'**il peut être assemblé à partir de deux ou de plusieurs segments.

13. Elément de garnissage empilable, selon l'une quelconque des revendications 1 à 12, **caractérisé en ce qu'**il est entièrement fabriqué dans une substance catalytiquement active ou qu'il est revêtu d'une substance catalytiquement active.

14. Utilisation d'un élément de garnissage empilable, comme défini dans la revendication 13, pour la pyrolyse de formylalaninenitrile ou de formamide.

15. Procédé pour la pyrolyse de formylalaninenitrile ou formamide dans un réacteur tubulaire, **caractérisé en ce que** la pyrolyse est réalisée en la présence d'un garnissage d'éléments de garnissage empilables, fabriqués en Al₂O₃, imprégnés de carbonate de potassium, comme définis dans l'une quelconque des revendications 1 à 13.

16. Garnissage d'éléments de garnissage empilés, comme définis dans l'une quelconque des revendications 1 à 13.

17. Réacteur tubulaire, colonne de distillation ou laveur de gaz, comprenant un garnissage selon la revendication 16.
